# EUROPEAN PATENT SPECIFICATION

(11) **EP 1 386 543 B1**
(45) Date of publication and mention of the grant of the patent: **17.08.2005**
(21) Application number: 02028532.6
(22) Date of filing: 20.12.2002
(51) Int. Cl.: A23K 3/03, A61K 31/198, C12R 1/25

(54) **New bacterial strain and the use thereof**
Neuer Bakterienstamm und seine Verwendung
Nouvelle souche bacterielle et utilisation

(30) Priority: 08.01.2002 SE 0104311
(43) Date of publication of application: 04.02.2004
(73) Proprietor: Medipharm AB, 260 23 Kageröd (SE)
(72) Inventor: Ström, Katrin, 754 24 Uppsala (SE); Sjögren, Jörgen, 754 24 Uppsala (SE); Magnusson, Jesper, 756 46 Uppsala (SE); Broberg, Anders, 752 67 Uppsala (SE); Schnürer, Johan, 756 47 Uppsala (SE)
(74) Representative: Karlsson, Leif

(56) References cited:
- EP-A- 0 687 673
- US-A- 4 528 199
- PATENT ABSTRACTS OF JAPAN vol. 2000, no. 13, 5 February 2001 (2001-02-05) -& JP 2000 300284 A (SEIBUTSU KASSEI KENKYUSHO:KK), 31 October 2000 (2000-10-31)
- DATABASE CA [Online] CHEMICAL ABSTRACTS SERVICE, COLUMBUS, OHIO, US; STROM, KATRIN ET AL: "Lactobacillus plantarum MiLAB 393 produces the antifungal cyclic dipeptides cyclo(L-Phe-L-Pro) and cyclo(L-Phe-trans-4-OH-L-Pro) and 3-phenyllactic acid" retrieved from STN Database accession no. 138:86202 XP002244399 & APPLIED AND ENVIRONMENTAL MICROBIOLOGY (2002), 68(9), 4322-4327 ,
- DATABASE CA [Online] CHEMICAL ABSTRACTS SERVICE, COLUMBUS, OHIO, US; JIANG, ZHONG ET AL: "Two diketopiperazines and one halogenated phenol from cultures of the marine bacterium, Pseudoalteromonas luteoviolacea" retrieved from STN Database accession no. 134:219489 XP002244400 & NATURAL PRODUCT LETTERS (2000), 14(6), 435-440 ,
- DATABASE CA [Online] CHEMICAL ABSTRACTS SERVICE, COLUMBUS, OHIO, US; CRONAN, JOHN M., JR. ET AL: "Plant growth promoters isolated from a marine bacterium associated with Palythoa sp." retrieved from STN Database accession no. 129:65348 XP002244401 & NATURAL PRODUCT LETTERS (1998), 11(4), 271-278 ,

## Description

The present invention refers to a new bacterial strain. More precisely, the present invention refers to a new bacterial strain which is intended to be used in connection with the preservation of straw or green fodder.

After harvest, straw or green fodder has generally been preserved by drying in the air and then storing under dry conditions. In modern agriculture new methods have been developed for preserving straw or green fodder under humid conditions. In these methods fresh fodder, which has just been harvested or slightly pre-dried, is stored in airtight envelopes. Lactobacilli naturally present in the straw or green fodder will then proliferate and produce lactic acid, which in turn provides an acid preservation of the fodder. However, the growth of lactobacilli under such conditions is relatively slow, and conditions are obtained which favor the growth of non-desired harmful microorganisms, for instance clostridia or mycotoxin producing fungi.

In order to improve and accelerate preservation effect various types of additives are mixed in the fresh fodder, for instance formic acid. This addition results in a decrease in the pH value of the straw or green fodder to about pH 4, which is the optimum pH value for preservation. Alternatively, or as a supplement to formic acid, molasses, lactobacilli, or certain enzymes have been added, which increase the availability of nutrients in the fodder. Chemical substances are also available on the market, which exhibit a direct preservation effect.

A disadvantage of only adding an acid in order to preserve a straw or green fodder is that other microorganisms than acid-producing bacteria may also develop.
In many cases these microorganisms are spore forming bacteria, generally referred to as clostridia. A high content of clostridia in an ensilage generally results in that the milk from a cow will also have a high content of clostridia. Since these bacteria are not killed in normal pasteurization processes, dairy products produced from such milk will also be negatively affected. For instance there may appear as a false fermentation in cheese products.

If airtight (anaerobic) conditions are not maintained in the silage growth of lactic acid degrading fungi and yeast may occur. This will result in spoilage, loss of nutrients as well as potential mycotoxin formation.

It is thus an object of the present invention to eliminate or reduce the disadvantages obtained in connection with conventional acidic preservation of straw or green fodder, and especially when conventional lactic acid bacteria (LAB) are used.

The object of the invention is also to provide compounds which are useful as preservation additives in ensilage, which inhibit the growth of harmful microorganisms in an ensilage, such as clostridia and mycotoxigenic fungi, and which does not promote fermentation in the ensilage.

Another object of the invention is to provide compositions that can be used for the preservation of straw or green fodder.

Thus, the present invention provides a biologically pure culture of a strain of a lactic acid bacterium, which has been selected for its ability to exhibit osmotolerance, for its ability to exhibit a suitable inhibition spectrum against fungi (fungi and yeast), and for its ability to produce antimicrobial compounds hitherto not produced by lactic acid producing bacteria.

The inventive bacterial strain, MiLAB 393, is a strain of *Lactobacillus plantarum.* This strain has been deposited on January 3, 2002, pursuant to, and in satisfaction of, the requirements of the Budapest Treaty on the International Recognition of the Deposit of Microorganisms for the Purposes of Patent Procedure at BCCMü/LMG Bacteria Collection, Universiteit Gent, K.L. Ledeganckstraat 35, B-9000 Gent, Belgium, under the deposition number LMG P-21295.

The LAB strain according to the invention can be prepared industrially by inoculating a pure culture of the same in a broth that has been sterilized in advance. The bacteria are then cultured for 10-20 hours at a temperature, which is the optimum temperature for the strain. The lactic acid formed in the culture is neutralized by the addition of for example NaOH in order to maintain the pH of the culture at an optimum value. When the density of the culture has reached a certain concentration the growth ceases and the bacterial cells are harvested. This is preferably accomplished as a concentration by means of a separator or a filter, the bacterial cells being separated from the culture broth.

*Lactobacillus plantarum*, strain MiLAB 393, is obtained in high yields and exhibit excellent stability.

The bacterial cells thus obtained are then desiccated, for example by means of freeze-drying. The powder of live bacterial cells can subsequently be suspended in water or another aqueous medium. The suspension is then supplied to the straw or green fodder by means of for instance spraying onto the same when it is put into packages or supplied to an ensilage tower. Of course, the harvested cells can also be used directly as such.

The inventive bacterial strain of *Lactobacillus plantarum* is thus used as a starter culture composition for ensiling straw or green fodder without air supply and subsequent anaerobic conditions. Such a composition kills or inhibits in the ensilage the growth of Gram-positive bacteria, yeast, fungi, and clostridia as well as certain Gram-negative bacteria. Furthermore, a good survival of the inventive LAB strain is obtained in the ensilage.

A composition for the preservation of straw or green fodder, which comprises *Lactobacillus plantarum, strain* MiLAB 393, can also comprise further lactic acid bacteria species as well as other ensilage additives. Preferably, *Enterococcus faecium* and/or *Pediococcus acidilacti* are included in the composition. These other lactic acid bacteria should be present in roughly equal amounts, and the total bacterial content should be 10⁵-10⁸ per gram of the composition. Preferably, *Lactobacillus plantarum*, strain MILAB 393, represents 10-90 % of the composition.

A composition, which comprises a biologically pure culture of the inventive *Lactobacillus plantarum* strain and optionally other lactic acid bacteria, may also include a suitable carrier. Such carriers are well known to the skilled man in the art.

The bacteria are preferably added in an amount of 4-8 liter per ton ensilage. When the ensilage is stored in a tower it should be finely chopped and have a dry matter (DM) content of 20-55%. *Lactobacillus plantarum*, strain MILAB 393, should represent 10-90 % of the composition.

Examples of ensilage additives to be used are molasses, enzymes that increase the availability of nutrients in the fodder, and preservation agents known within the art, which exhibit a direct preservation effect. Preferably, the enzyme is a fodder degrading enzyme, e.g. a cellulase.

*Lactobacillus plantarum*, strain MiLAB 393, exhibits a high osmotolerance. This means that the inventive LAB strain is able to grow as well as produce antimicorbial compounds in an ensilage system having a dry matter content of more than 45%. Strain MiLAB 393 is able to inhibit the growth of both yeast and fungi. An excellent inhibition is exhibited towards for example *Aspergillus fumigatus,* Peni*cillium commune, Rhodotorula mucilaginosa, Kluyveromyces marxianus*, and *Candida albicans.*

Apart from producing adequate amounts of lactic acid, *Lactobacillus plantarum*, strain MiLAB 393, produces other antagonistic substances. Three compounds have been isolated from growth media of the inventive LAB strain, which exhibit antagonistic effects towards yeast and fungi. These compounds have been identified as cyclo-(L-phenylalanine-*trans*-4-hydroxy-L-proline), cyclo-(L-phenylalanine-L-proline), and 3-phenyl-lactic acid, respectively. The antimicrobial activity of 3-phenyl-lactic acid can be attributed to about 90% 3-phenyl-(D)-lactic acid and about 10% 3-phenyl-(L)-lactic acid.

Thus, a cell-free culture supernatant obtained from a biologically pure culture of *Lactobacillus plantarum*, strain MiLAB 393, can be used as such as an antimicrobial agent in order to inhibit the growth of harmful microorganisms in straw or green fodder. Of course, the supernatant can be divided into fractions containing one or antimicrobial compounds produced.

### EXAMPLES

The following examples will further describe and illustrate the invention.

### Example 1. Strain isolation and strain identification

The inventive strain was isolated from the epiphytic flora in a grass silage experiment, in which the aerobic stability after the addition of various antifungal LAB was evaluated. More specifically, strain MiLAB 393 was isolated from one of three control silos since they exhibited the most efficient inhibition of fungal growth. Ten gram of silage material was suspended in 90 ml sterile peptone water (0.2% [w/v]) and mixed in a Stomacher® sample blending device. Dilutions were prepared by using sterile peptone water and were surface spread on MRS (Man, Rogosa and Sharpe) agar plates. After anaerobic incubation at 30°C for 48 hours the bacterial cultures were transferred to fresh MRS agar plates for a second incubation.

The identity of the bacterial strain MiLAB 393 was established both by its fermentation pattern and by 16S rDNA sequencing. An AP1 50 CHL test (BioMérieux) showed a 99.9% identity with *Lactobacillus plantarum.*

For 16S rDNA sequencing, chromosomal DNA was isolated by using the Dneasy™ Tissue Kit (QIAGEN). PCR amplification (94°C for 30 s, 54°C for 30 s, and 72°C for 80 s, 30 cycles) and by using primers 16S.S (5'-AGAGTTTGATCCTGGCTC-3') and 16S.R (5'-CGGGAACGTATTCACCG-3') amplified approximately 1400 bp of the 16S ribosomal DNA. Sequencing of the amplifred DNA was performed by using the Thermo Sequenase™ dye terminator cycle sequencing pre-mix (Amersham) and the automated sequence analyzer AB1 PRISM 377XL (Perkin Elmer). Partial 16S rDNA sequence was used for searching in public databases and the identity of the isolated strain MiLAB 393 as *Lactobacillus plantarum* was confirmed.

### Example 2. Antimicrobial inhibition spectrum

The inhibition spectrum of strain MiLAB 393 was determined by using an overlay method, in which bacterial streaks on a MRS plate were overlaid with soft agar containing fungal spores. After 48 h of anaerobic growth at 30°C (BBL®GasPak *Plus*™) the bacterial streaks were overlaid with soft agar (1% agar) containing 0.05 % malt extract (Oxoid LDT, Basingstoke, Hampshire, England) and 10⁴ spores/ml (10⁴ cells/ml) of the fungus or yeast to be tested. After 48 h of aerobic incubation at 30°C the inhibition zone was measured. The grade of inhibition was determined by relating the inhibited growth area per inoculation streak to the total area of the Petri dish. The inhibition was graded as follows: -, no visible inhibition; (+), indicates a weak inhibition in the soft agar above the streak; +, inhibition area per bacterial streak 0.1-3.0 %; ++, inhibition area per bacterial streak 3.0-8.0 %; or +++, inhibition area per bacterial streak >8.0 %.

A table of the inhibition spectrum of MiLAB 393 obtained by means of the overlay method is shown below.

| Fungus/Yeast | Inhibition |
|---|---|
| *Aspergillus fumigatus* | +++ |
| *Penicillium roqueforti* | - |
| *Penicillium commune* | ++ |
| *Aspergillus nidulans* | ++ |
| *Pichia anomala* | (+) |
| *Rhodotorula mucilaginosa* | ++ |
| *Saccharomyces cerevisiae* | (+) |
| *Kluyveromyces marxianus* | +++ |
| *Candida albicans* | ++ |
| *Zygosaccharomyces bailii* | - |
| *Debaromyces hansenii* | ++ |

### Example 3. Purification and identification of antimicrobial compounds

*Lactobacillus plantarum*, strain MiLAB 393, was inoculated to a concentration of 10⁵ in 2000 ml of MRS broth as a still culture at 30°C for 48 h. A cell free supernatant was prepared by means of centrifugation (7000 rpm for 15 min) and sterile filtration (45 µm, Millipore®). The cell free culture filtrate was used for further purification of the antimicrobial compounds. All fractions in the purification process concentrated by means of lyophilization. Then they were evaluated for any antimicrobial activity by using a microtiter plate well assay with *Aspergillus furnigatus* as an indicator strain.

The isolation of antifungal compounds was performed by using C18 SPE (Isolute C18 EC, 10 g/70 ml), C18-HPLC (Discovery C18, 100x21.2 mm, 5µ Supelco) and Hypercarb-HPLC (Hypersil 100x21.2 mm, 5µ). The system used was a preparative HPLC system (Gilson pump 305 and 306) connected to an UV-detector (Gilson 118); flow: 10 ml/min; detection: 210 nm; and integrator: Merck Hitachi D-7500.

Antimicrobial compounds were isolated after three rounds of separation:
1. The supernatant was separated into two phases on a C18-SPE column with 95% acetonitrile (ACN) as a solvent.
2. The ACN fraction from step 1 was separated on a C18-HPLC column (an aacetonitrile/water gradient from 5% ACN to 100% ACN for 10 min; then held 5 min at 100%).
3. Active fractions from step 2 were further separated on a Hypercarb HPLC-column (mobile phase: 40% ACN/60% 0.1% trifluoroacetic acid in water).

### Example 4. Structural determination

NMR spectra were recorded on a Bruker DRX-600 spectrometer (600 MHz proton frequency) equipped with a 5 mm probe. Compounds (4-10 mg/ml) were dissolved in CD₃OD or deuturated dimethyl sulfoxide at a temperature of 30°C. A Bruker standard pulse sequence was used. For the two diketo-piperazines 1H, COSY, HMBC and HMQC were performed and for phenyl-lactic acid only 1H and HMBC. Molecular weights were determined by mass spectroscopy by using an ESI-Ion Trap MS (Esquire-LC, Bruker Daltoniks, GmbH). The samples were dissolved in methanol, about 0.4 pg/ml.

The following structures were obtained for the compounds with antimicrobial activity, which had been isolated from *Lactobacillus plantarum*, strain MiLAB 393: 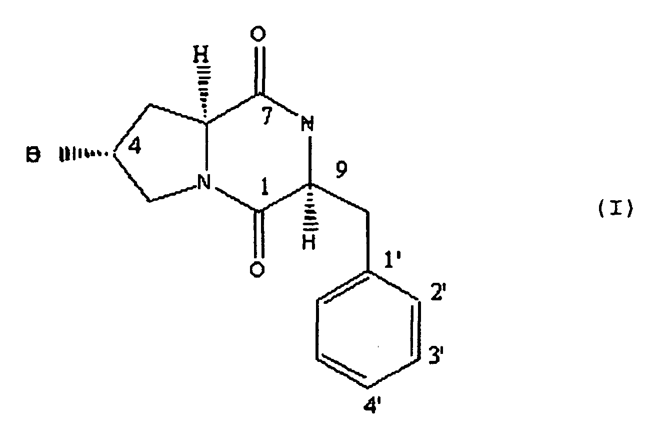 cyclo-(L-phenylalanine-trans-4-hydroxy-L-proline) Systematic name: (3S,7R,9S)-hexahydro-7-hydroxy-3-(phenylmethyl)-pyrrolo(1,2-a)pyrazine-1,4-dione 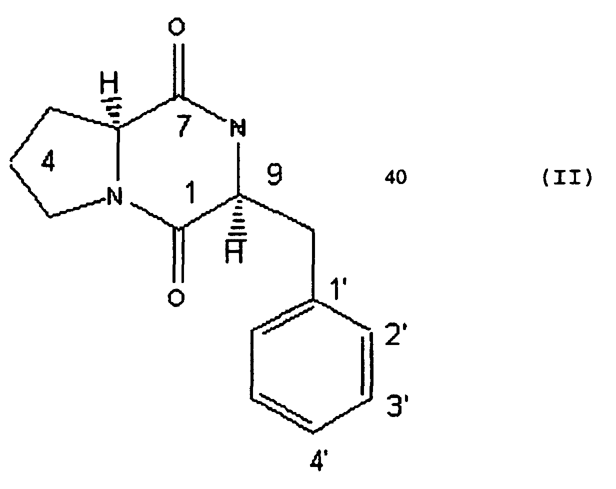 Cyclo(L-phenylalanine-L-proline) Systematic name: (3S,9S)-hexahydro-3-(phenylmethyl)-pyrrolo(1,2-a)pyrazine-1,4-dione 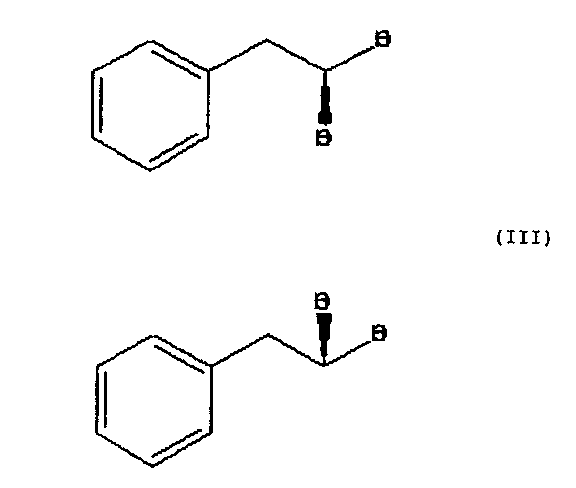 3-Phenyl-lactic acid

### Example 5. Osmotolerance of strain MiLAB 393

The osmotolerante of the inventive strain was determined by dilution plating on MRS agar plates containing 10% KCl (corresponding to a water activity a_{w} of about 0.93 and a dry matter content of 45% (DM/kg). As a result, the numbers of MiLAB 393 CFU (colony forming units) formed on agar plates containing KCl was very close to that formed on MRS agar plates without KCl as shown below.

| **CFU on MRS** | **CFU on MRS + 10% KCL** |
|---|---|
| 3.0*10⁹ | 2.8*10⁹ |

### Example 5. Comparation of osmotolerance

In this osmotolerance trial on grass three different dry-matters, 30, 45 and 55 %, were used to evaluate according to standard procedures the osmotolerance properties of two batches of strain MiLAB 393 (Batch A and Batch B) as well as one batch (Batch C) of *Lactobacillus plantarum,* strain *LSI*, which exhibits excellent osmotolerance and which hitherto had had been the preferred strain with reference to this property. The dry-matter percentages are all measured on the chopped fresh matter.

### Initial pH decrease

The pH decrease in low DM grass, 30 %, was measured twice a day after ensiling, since a fast decrease was expected. The pH did drop quickly, and after the first day there is a significant (p ≤ 0.05) difference between the treatments, the bacteria of Batch A being a bit slower than those of Batch B and Batch C after 20 hours. After two days the pH had reached 4,5 with all the three treatments. After three days, there is no significant difference between the treatments.

In the case of pH decrease in DM grass, 45%, the decrease in pH in this medium DM silage was not as fast as in the low DM silage. Treatment with Batch A resulted in a lag of the initial decrease in pH. After 2 days the difference was significant (p ≤0.05), but this significance between the treatments was lost after three days. Once again, Batch A seems to be a bit inferior when it comes to quickly acidifying the crop while there is no difference in the Batch C and Batch B treatments when ensiling intermediate DM grass.

The pH in high DM grass, 55%, decreased slowly and did not reach acceptable levels until after eight days of ensiling. However, it should be noted that a low pH around 4-4.5 is not necessary in order to produce well-preserved silage. A pH just below 5.0 is acceptable.

In the initial pH decrease there is no difference between the treatments. After two days ensiling there is a significant (p ≤0,05) difference between all the treatments, Batch B being the superior. By day five there is a significant difference between Batch B and Batch C, but not between Batch A and either of the other treatments. After eight days Batch C was significantly inferior to the treatments with Batch A and Batch B.

Thus, both batches with *Lactobacillus plantarum*, strain MiLAB 393, seem to be well suited for ensiling high DM crops.

### Microbial analysis

A microbial evaluation was performed on the high DM grass after ensilation, the yeast and fungi counts in high DM grass being determined after 12 days.

Low counts were obtained for Batch A and Batch B, and a rather high value was obtained for Batch C.

Thus, treatments with *Lactobacillus plantarum*, strain MiLAB 393, seem to have an effect on yeast and fungi, which the bacteria of Batch C lack.

## Claims

1. A biologically pure culture of *Lactobacillus plantarum*, strain MiLAB 393 "LMG P-21295".

2. A composition for the preservation of straw or green fodder, which comprises a biologically pure culture of *Lactobacillus plantarum*, strain MILAB 393 LMG P-21295, and a suitable carrier.

3. The composition as in claim 2, **characterized in that** it further comprises *Enterococcus faecium* and/or *Pediococcus acidilacti.*

4. The composition as in claim 2 or 3, **characterized in that** it further comprises a fodder degrading enzyme.

5. The composition as in any of claims 2-4, **characterized in that** *Lactobacillus plantarum*, strain MILAB 393 LMG P-21295, represents 10-90 % of the same.

6. Use of a composition as in any of claims 2-5 for the preservation of straw or green fodder, **characterized in that** *Lactobacillus plantarum,* strain MILAB 393 LMG P-21295, is added to fresh straw or green fodder in an amount of 10⁴-10⁶ per gram of fodder.

7. A cell-free culture supernatant, or a fraction thereof, which is obtained from a biologically pure culture of *Lactobacillus plantarum,* strain MILAB 393 LMG P-21295.

8. Use of a cell-free culture supernatant, or a fraction thereof, which is obtained from a biologically pure culture of *Lactobacillus plantarum,* strain MILAB 393 LMG P-21295, as an antimicrobial agent to inhibit growth of harmful microorganisms in ensilage.

9. Use as in claim 8, wherein the antimicrobial agent is 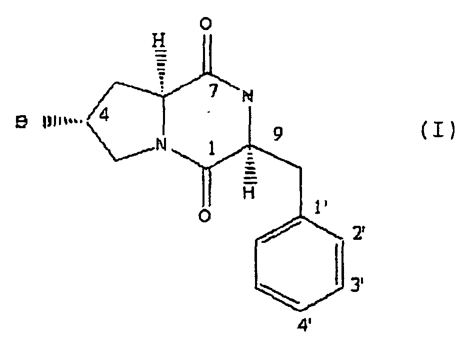 cyclo-(L-phenylalanine-trans-4-hydroxy-L-proline).

10. Use as in claim 8, wherein the antimicrobial agent 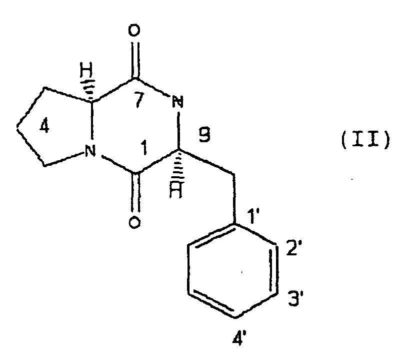 Cyclo(L-phenylalanine-L-proline).

11. Use as in claim 8, wherein the antimicrobial agent is 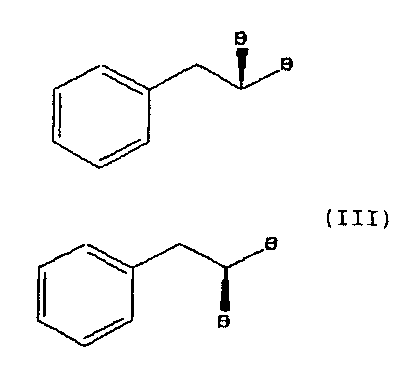 3-Phenyl-lactic acid.

## Patentansprüche

1. Biologische Reinkultur von Lactobacillus plantarum, Stamm MiLAB 393 LMG P-21295.

2. Zusammensetzung zum Haltbarmachen von Stroh oder Grünfutter, die eine biologische Reinkultur von Lactobacillus plantarum, Stamm MiLAB 393 LMG P-21295 und einen geeigneten Träger umfasst.

3. Zusammensetzung nach Anspruch 2, **dadurch gekennzeichnet, dass** sie ferner Enterococcus faecium und/oder Pediococcus acidilacti umfasst.

4. Zusammensetzung nach Anspruch 2 oder 3, **dadurch gekennzeichnet, dass** sie femer ein Futter zersetzendes Enzym umfasst.

5. Zusammensetzung nach einem der Ansprüche 2 bis 4, **dadurch gekennzeichnet, dass** Lactobacillus plantarum, Stamm MiLAB 393 LMG P-21295 10 - 90 % derselben darstellt.

6. Verwendung einer Zusammensetzung nach einem der Ansprüche 2 bis 5 zum Haltbarmachen von Stroh oder Grünfutter, **dadurch gekennzeichnet, dass** Lactobacillus plantarum, Stamm MiLAB 393 LMG P-21295 frischem Stroh oder Grünfutter in einer Menge von 10⁴ - 10⁶ pro Gramm Futter zugegeben wird.

7. Zellfreier Kulturüberstand oder eine Fraktion davon, der/die aus einer biologischen Reinkultur von Lactobacillus plantarum, Stamm MILAP 393 LMG P-21295 erhalten wird.

8. Verwendung eines zellfreien Kulturüberstands oder einer Fraktion davon, der/die aus einer biologischen Reinkultur von Lactobacillus plantarum, Stamm MiLAB 393 LMG P-21295 erhalten wird, als antimikrobieller Wirkstoff, um die Vermehrung schädlicher Mikroorganismen in der Silage zu hemmen.

9. Verwendung nach Anspruch 8, wobei der antimikrobielle Wirkstoff 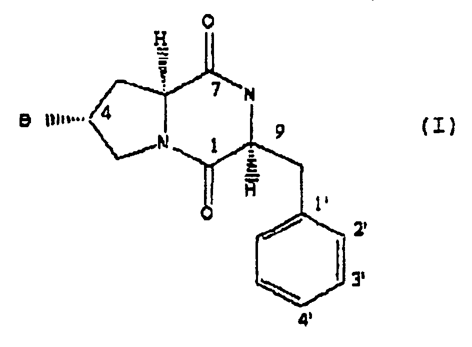 Cyclo-(L-phenylalanin-trans-4-hydroxy-L-prolin) ist.

10. Verwendung nach Anspruch 8, wobei der antimikrobielle Wirkstoff 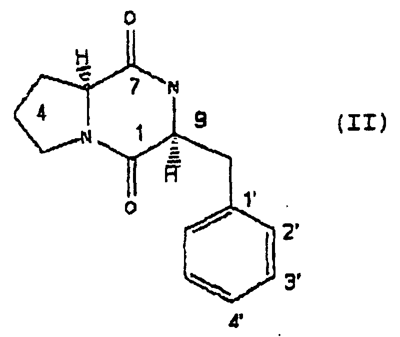 Cyclo-(L-phenylalanin-L-prolin) ist.

11. Verwendung nach Anspruch 8, wobei der antimikrobielle Wirkstoff 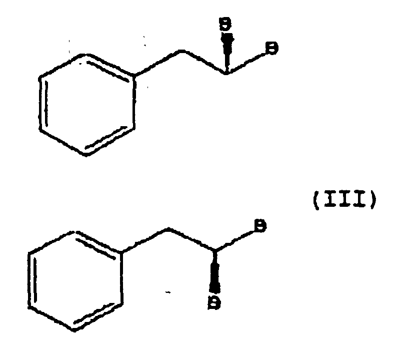 3-Phenylmilchsäure ist.

## Revendications

1. Culture biologiquement pure de *Lactobacillus plantarum,* souche MiLAB 393 "LMG P-21295".

2. Composition pour la préservation de la paille ou du fourrage vert, qui comprend une culture biologiquement pure de *Lactobacillus plantarum*, souche MILAB 393 LMG P-21295, et un porteur adapté.

3. Composition selon la revendication 2, **caractérisée en ce qu'**elle comprend en outre *Enterococcus* faecium et/ou *Pediococcus acidilacti.*

4. Composition selon la revendication 2 ou 3, **caractérisée en ce qu'**elle comprend en outre une enzyme de dégradation du fourrage.

5. Composition selon l'une quelconque des revendications 2 à 4, **caractérisée en ce que** *Lactobacillus plantarum*, souche MILAB 393 LMG P-21295, représente 10 à 90 % de celle-ci.

6. Utilisation d'une composition selon l'une quelconque des revendications 2 à 5 pour la préservation de la paille ou du fourrage vert, **caractérisée en ce que** *Lactobacillus plantarum*, souche MILAB 393 LMG P-21295, est ajouté à de la paille fraîche ou du fourrage vert dans une quantité allant de 10⁴ à 10⁶ par gramme de fourrage.

7. Surnageant de culture dépourvu de cellule, ou une fraction de celui-ci, qui est obtenu à partir d'une culture biologiquement pure de *Lactobacillus plantarum*, souche MILAB 393 LMG P-21295.

8. Utilisation d'un surnageant de culture dépourvu de cellule, ou une fraction de celui-ci, qui est obtenu à partir d'une culture biologiquement pure de *Lactobacillus plantarum*, souche MILAB 393 LMG P-21295, comme agent antimicrobien pour empêcher le développement de microorganismes nuisibles dans l'ensilage.

9. Utilisation selon la revendication 8, dans laquelle l'agent antimicrobien est 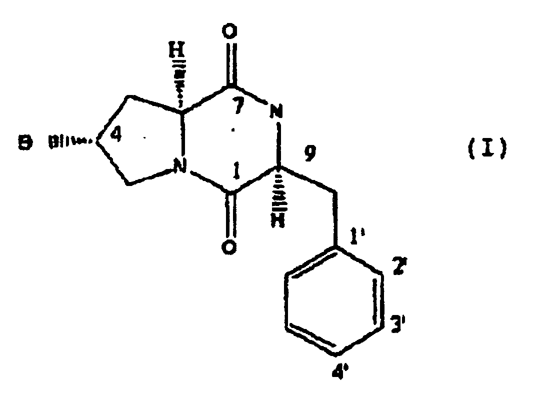 cyclo-(L-phénylalanine-trans-4-hydroxy-L-proline).

10. Utilisation selon la revendication 8, dans laquelle l'agent antimicrobien est 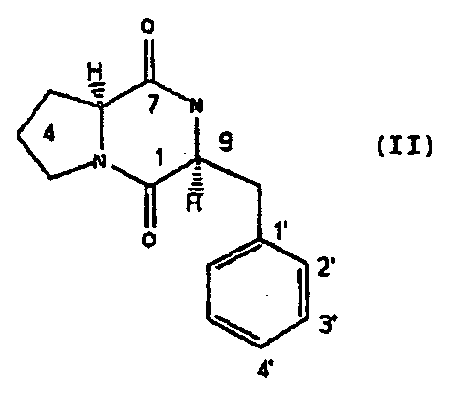 cyclo(L-phénylalanine-L-proline).

11. Utilisation selon la revendication 8, dans laquelle l'agent antimicrobien est 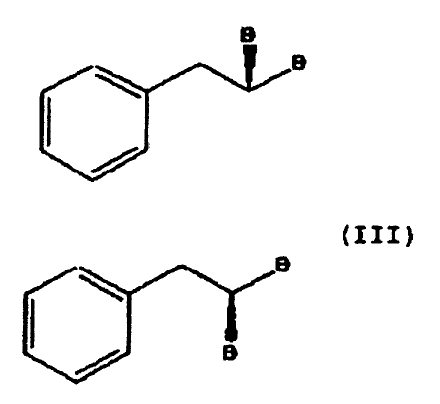 3-phényle-acide lactique.
